Europäisches Patentamt

(19) European Patent Office

Office européen des brevets

(11) Publication number: **0 235 754**
**A2**

## (12) EUROPEAN PATENT APPLICATION

(21) Application number: 87102757.9

(22) Date of filing: 26.02.87

(51) Int. Cl.4: **C12N 15/00** , C07K 13/00 , C07K 15/04 , C12N 1/20 , C12Q 1/70 , A61K 39/15 , C12P 21/00 , //(C12N1/20,C12R1:19)

(30) Priority: 04.03.86 US 836005

(43) Date of publication of application: 09.09.87 Bulletin 87/37

(84) Designated Contracting States: BE DE FR IT

(71) Applicant: **ABBOTT LABORATORIES**
**14th Street and Sheridan Road North St**
**North Chicago Illinois 60064(US)**

(72) Inventor: **Smith, Robert E.**
**36567 N. Grandwood Drive**
**Gurnee Illinois 60031(US)**

(74) Representative: **Modiano, Guido et al**
**MODIANO, JOSIF, PISANTY & STAUB**
**Modiano & Associati Via Meravigli, 16**
**I-20123 Milan(IT)**

(54) **Composition and methods of use for a lacZ rotavirus fusion protein.**

(57) SA-11 rotavirus major inner capsid protein (VP6) was cloned and expressed in E. coli as a fusion protein. The fusion protein is formed by nine amino acids resulting from expression of a portion of the 5'-noncoding region immediately upstream of the lacZ gene linked to the six N-terminal amino acids of B-galactosidase in turn linked to rotavirus SA-11 major inner capsid protein VP6. The complete fusion protein and 27K and 24K fragments thereof (also referred to as fusion proteins according to the present invention) exhibited greater antigenicity than the native protein.

FIG. 1

## COMPOSITIONS AND METHODS OF USE FOR A LacZ ROTAVIRUS FUSION PROTEIN

### Background Of The Invention

The present invention pertains in general to viral proteins and assays and vaccines employing them and in particular to fusion proteins related to the rotavirus (SA-11 strain) major core antigen (gene 6).

Human rotavirus has been shown in recent years to be the principal causative agent of diarrhea in infants and young children. Epidemiological research suggests that rotaviruses are ubiquitous in nature and constitute a major health problem worldwide, particularly in underdeveloped countries where combined with the effects of malnutrition, they can cause chronic illness and death.

Rotaviruses are members of the virus family Reoviridae and are characterized by double-stranded RNA genomes arranged in eleven segments. Kapikian et al. "Rotaviruses," Chapter 37 in Virology, pp. 863-906, Fields ed., Raven Press, New York (1985). Ultrastructural analyses show that rotavirus gene segments are packaged in a core particle surrounded by inner capsid and outer capsid layers. Palmer et al., J. Gen. Virol., 35, 403-414 (1977); and Palmer et al., J. Gen. Virol., 62, 105-111 (1982). The proteins comprising the inner and outer capsid are now well characterized, particularly for the simian rotavirus SA-11, and gene coding assignments exist for most of the proteins. Kapikian et al., supra.

Almost all mammalian rotaviruses are serologically related and fall into two groups which may be distinguished antigenically by complement fixation, enzyme linked immunosorbent assay (ELISA) and Immune Adherence Hemagglutination tests. These group specific antigens are mapped to the major inner capsid protein (VP6) of rotavirus coded for by RNA segment 6 (gene 6) [Kapikian et al., Infec. Immun., 33, 415-425 (1981); and Kalica et al., Virology, 112, 385-390 (1981)]. Antibodies directed against rotavirus inner capsid particles (predominantly VP6) constitute the basis for the Rotazyme® test (Abbott Laboratories, North Chicago, Illinois) and are used in other ELISA and agglutination assays as reliable markers for rotavirus infection.

Monoclonal antibodies which react specifically to the major inner capsid proteins of several rotavirus isolates are used to identify both common and group specific epitopes which map to the VP6 protein, Greenberg et al, Infec. and Immun., 39, 91-99 (1983). Monoclonal antibodies directed against VP6 are also used as reagents in ELISA tests for detection of rotavirus.

The majority of animal rotavirus isolates have been classified as belonging to serogroup 1, while most human rotaviruses fall into the group 2 category. However, both serogroups are represented in human and animal rotavirus strains. Some of the more extensively studied isolates are bovine (NCDV, group 1; UK, group 1), equine (H-1, group 1), porcine (OSU, group 1), canine (CU-1, group 1), simian (SA-11, group 1), and human (Wa, group 2; S2, group 1, M; group 2).

Complementary DNA (cDNA) copies of RNA segment 6 from strains representing both group 1 (SA-11) and group 2 (Wa) serogroups may be synthesized and cloned in plasmid vectors in E. coli. G.W. Both, et al., Nucleic Acids Res., 10, 7075-7088 (1982); Estes et al., Nucleic Acids Res., 12, 1875-1887 (1984); and Both et al., J. Virol. 51, 97-101 (1984). Comparison of gene 6 DNA sequences indicates that most of the genome is highly conserved with a few variable regions which may account for differences in subgroup specificity, Both et al., J. Virol., 51, 97-101 (1984).

SA-11 gene 6 may be expressed as a fusion protein in E. coli using the lac promoter. Smith et al., Annual Meeting of the American Society for Microbiology, March 3-7, 1985, Abstract #T13 (presented March 4,1985). The SA-11 gene 6 may be expressed, alternately, as a native protein in E. coli under control of the lambda $P_L$ promoter. Bellamy et al., ASV Annual Meeting, Workshop #30, July 25, 1984. SA-11 gene 6 may also be expressed in a nuclear polyhedrosis virus (NPV) -insect cell expression system. [Summers, First Annual Southwest Foundation for Biomedical Research International Symposium, November 8-10, 1984].

### Summary Of The Invention

The present invention provides a fusion protein more active than the native major inner capsid protein in a test of antigenic reactivity. The fusion protein may be (a) a lacZ gene product-rotavirus major inner capsid fusion protein; or (b) a lacZ gene product-rotavirus major inner capsid fusion protein further including a 5′-noncoding region of the lacZ gene; or (c) a fusion protein having an amino acid sequence as set forth in Table 1; or (d) a fragment of (a), (b), or (c) and in particular, fragment having molecular weights of about 27K, or 24K as determined by electrophoretic mobility on a 10 percent SDS-acrylamide gel. The present invention also provides nucleic acids encoding fusion proteins according to the present invention, and host cells, preferably protease-deficient host cells, capable of expressing the fusion proteins according to the present invention and transformed with a nucleic acid according to the present invention.

An assay for detecting the presence of human antibodies against a rotavirus in a sample according to the present invention includes the step of exposing a sample of antibody-containing material in a test solution to a fusion protein according to the present invention. The fusion protein is bound to a support, reacts immunologically with antibody material in the sample, and immobilizes the antibody material on the support. A second antibody, capable of specifically binding with the antibody in the sample and associated with a reporter group or label (e.g., an enzyme label, a fluorescent label or other labels conventional in the art) is introduced into the test solution. The presence of reporter group bound to the support indicates the presence of antibody against rotavirus in the sample.

A vaccine according to the present invention includes an immunologically acceptable diluent, adjuvant or carrier and a fusion protein according to the present invention.

A method of raising polyclonal antibodies according to the present invention includes the steps of immunizing an animal with an effective amount of a fusion protein according to the present invention, and isolating anti-VP6 antibodies from serum of the animal. The present invention also provides antibodies raised according to this method.

A method of producing monoclonal antibodies according to the present invention includes the steps of immunizing an animal against a fusion protein according to the present invention, fusing antibody-producing cells from the animal to myeolma cells; isolating a cell line producing a monoclonal antibody against VP6 in a medium, and purifying monoclonal antibody against VP6 from the medium. The present invention also provides monoclonal antibodies produced by this method.


Brief Description Of The Drawings

Figure 1 is a schematic depiction of the construction of a library of cDNA according to the present invention;

Figure 2 is a partial restriction map of a rotavirus SA-11 gene 6 cDNA according to the present invention;

Figure 3 is a comparison of a nucleic acid sequence for rotavirus SA-11 gene 6 according to the present invention with published sequences;

Figure 4 is a comparison of an amino acid sequence for rotavirus SA-11 major inner capsid protein - (VP6) according to the present invention with a published sequence; and

Figure 5 is a schematic illustration of the construction of a fusion protein according to the present invention.


Detailed Description

In accordance with the present invention, rotavirus SA-11 RNA gene segments were used as templates to synthesize DNA copies which were subsequently cloned into plasmid vectors in E. coli . A cloned DNA copy of SA-11 gene 6 was identified and verified by DNA sequence analysis to contain the entire protein coding sequence of the major inner capsid protein (VP6).

SA-11 gene 6 cloned DNA was expressed under control of the lac promoter as a fusion protein in vitro in an E. coli cell free system and in vivo using the protease-deficient E. coli strain WM 6. The resulting expressed gene products have been shown to be immunogenic by reacting with polyclonal antisera derived against rotavirus particles and mono-specific antisera derived against purified VP6 by immune-precipitation and Western blot assays.

These E. coli - expressed SA-11 gene 6 protein products may be seen to have utility as diagnostic reagents to detect antibodies to rotavirus in clinical samples and as immunogens for production of polyclonal or monoclonal antibodies to the rotavirus major surface antigen. Expression of SA-11 gene 6 in E.coli also allows for modification of the antigenic determinants of the recombinant VP6 protein product by site specific mutagenesis of the cloned gene 6 DNA.

In the following examples, restriction endonucleases were supplied by New England Biolabs (NEB). DNA polymerase Klenow fragment, polyA polymerase, and DNA ligase were purchased from Bethesda Research Labs (BRL) Gaithersburg, Maryland. Reverse transcriptase was supplied by Life Sciences. RNA ligase and terminal transferase were supplied by Pharmacia Molecular Biologicals (PMB). $^{32}P$-labelled reagents, including nick translation reagents, were purchased from New England Nuclear (NEN). $^{35}S$-labelled reagents, including E. coli DNA-directed transcription/translation kits were purchased from Amersham. Agarose, acrylamide, and other electophoresis reagents were supplied by Biō Rad. Growth media were obtained from Difco. X-gal was purchased from BRL. Media and buffers for growth of SA-11 rotavirus were obtained from Grand Island Biological Company (GIBCO).

E. coli strains JM 83, JM 103, and JM 105 were obtained from PMB. Plasmids for cloning and expression (pUC 8, pUC 9) were purchased from PMB. M13 vectors (mp8) and primers for Sanger sequencing were purchased from PMB.

E. coli strain WM 6 was developed and is available from Abbott Laboratories, North Chicago, Illinois as a derivative of CAG456 which carries a nonsense mutation in the htpR (rpoD) gene encoding the RNA polymerase sigma subunit specific for heat shock promoters. The mutation may be suppressed, and proteolysis restored, by a temperature sensitive suppressor gene when cells are grown at low temperature - [Baker et al., Proc. Nat'l. Acad. Sci. (USA), 81, 6779-6783 (1984)]. Strain WM6 was isolated as a revertant of CAG456 which can grow at higher temperatures, up to 42° C. The proteolytic defect is maintained in this WM6 strain (A. Goldberg, personal communication). An advantageous feature of strain WM 6 is the lack of a need for heat induction to reduce proteolysis and the potential for the accumulation of of expressed protein over a longer period of time.

Standard procedures were employed for: plasmid purification, transformation of E. coli, small scale plasmid preparation, analyses of DNA by agarose gel electrophoresis, screening of recombinant colonies using X-gal, and colony hybridization, [Maniatis et al., Molecular Cloning -A Laboratory Manual, Cold Spring Harbor Laboratory, Cold Spring Harbor, New York (1982)]; and for analyses of proteins by SDS-acrylamide gel electrophoresis (PAGE) [Laemmli, Nature, 227, 680-685 (1970)]. Restriction enzymes (NEB), RNA ligase (PMB), poly A polymerase (BRL), terminal transferase (BRL) DNA polymerase Klenow fragment (BRL), and DNA ligase (BRL) were used according to the manufacturers' recommendations.

The following examples set forth the present invention in greater detail. Example 1 describes the synthesis of rotavirus SA-11 cDNAs and cloning thereof in E. coli. Example 2 details the identification and mapping of SA-11 gene 6 cloned DNA. In Example 3, the results of a Northern blot analysis of SA-11 gene 6 cloned DNA are presented. Example 4 describes a sequence analysis of SA-11 gene 6 cloned DNA. In Example 5, a description of the subcloning of SA-11 gene 6 DNA for expression in E. coli is provided. Example 6 details expression of SA-11 gene 6 cloned in vitro in an E. coli cell-free system. Example 7 provides a demonstration of antigenicity of SA-11 gene 6 cloned DNA products as expressed in vitro in an E. coli cell-free system. In Example 8, SA-11 gene 6 cloned DNA is expressed in vivo in E. coli. Example 9 sets forth the benefit attending expression of SA-11 gene 6 cloned DNA in an E. coli protease-deficient strain (WM 6). Example 10 describes a solid phase assay according to the present invention for the detection of human antibodies against rotavirus in a human stool or serum sample. Example 11 provides an enzyme-linked immunosorbent assay according to the present invention. In Example 12, a vaccine according to the present invention is described. In Example 13, polyclonal and monoclonal antibodies are produced according to the present invention.

## Example 1

### Propagation and Purification of SA-11 Rotavirus

SA-11 rotavirus [ATCC No. VR899] was propagated by infection of confluent MA104 cells (MA. Bioproducts) in roller bottles as indicated by Estes et al., J. Virol., 31, 810-815 (1979), except that tryptose phosphate and gentamicin were omitted from the media, and phosphate buffered saline (PBS, available from GIBCO) was used as a wash buffer instead of Tris buffered saline (TBS). Virus was propagated in the absence of trypsin or fetal calf serum and harvested after two days growth at 37° C. SA-11 stock virus was activated prior to infection by treatment with a 1/100 volume 10X tissue culture grade trypsin (GIBCO) for one hour at room temperature. $^{35}S$-labelled SA-11 virus was grown in a similar manner except methionine-free Minimal Eagles Medium (MEM) (GIBCO) was used along with 15 microcuries per milliliter ($\mu$Ci/ml) $^{35}S$-L-methionine (Amersham). Virus was purified by freeze-thawing infected cells two times followed by centrifugation for 20 minutes at 5000g and at 4° C to remove cellular debris. Eight percent (w/v) polyethylene glycol 6000 (PEG) was added to the clarified supernatant and allowed to stand overnight at 4° C. The media was then centrifuged 20 minutes at 5000g (4° C) to pellet PEG-precipitated SA-11 virus. The pellet containing virus was solubilized in a small volume of 50 mM Tris-HCl (pH 7.5) buffer and applied to a step gradient consisting of 1.4 g/ml CsCl, 1.3 g/ml CsCl, and 45% (w/v) sucrose in Tris buffer (20° C). Samples were centrifuged in a swinging bucket rotor for 3-6 hours at 100,000g (20° C). Virus particles, seen as a sharp bluish band migrating between 1.4-1.3 g/ml CsCl, were harvested and dialyzed overnight against 50 mM Tris-HCl (pH 7.5). Dialyzed virus served as a purified source material for isolation of genomic double-stranded RNA (dsRNA) or synthesis of virally coded messenger RNA (mRNA).

Isolation of SA-11 dsRNA and [32P]-end Labelling

Double-stranded RNA was extracted from CsCl-purified SA-11 virus by addition of an equal volume of Tris-HCl (pH 7.5) buffer-saturated phenol followd by vortexing (phenol extraction). Double-stranded RNA contained in the aqueous phase was concentrated by precipitation with 0.2M sodium acetate (NaAc) and two volumes of ethanol then stored at -20° C until use.

SA-11 dsRNA was end-labelled to serve as a size marker when resolving polyadenylated dsRNA by agarose gel electrophoresis or for Northern blot analysis of SA-11 dsRNA segments resolved by acrylamide gel electrophoresis.

Double-stranded RNA was labelled by addition of [32P]-Cp (cytosine 5′, 3′-diphosphate) to the 3′-termini of the RNA strands using RNA ligase as described by the manufacturer (PMB).

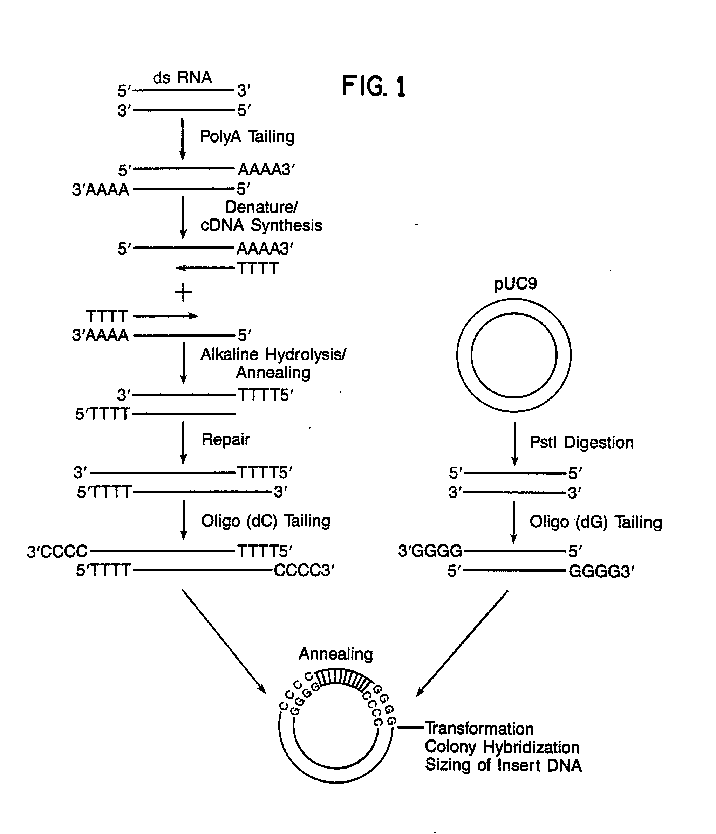

As shown in Fig. 1, a mixture of 11 segments of SA-11 double-stranded RNA (dsRNA) was isolated from SA-11 virus particles.

Polyadenylation of SA-11 dsRNA

Adenosine residues were added to the 3′-termini of SA-11 dsRNA (polyA-tailed dsRNA) to provide a template binding site for cDNA synthesis using reverse transcriptase. Standard methods were used (i.e., as recommended by BRL) except that 2.5 U/$\mu$l of RNasin (Promega Biotech) was added as a nuclease inhibitor. Five to ten micrograms (ug) of dsRNA were polyadenylated in a two minute reaction at 37° C resulting in the addition of 20-40 adenosine residues. [3H]-ATP was usually included at 10,000 Ci/mol in the reactions to measure RNA concentrations. Occasionally, ($\alpha$-[32P]) ATP (10,000 Ci/mol) was added for size analysis of polyadenylated (poly A) dsRNA by electrophoresis on a one percent agarose gel. [3H]-labelled poly A dsRNA was stored in 70% ethanol (-20° C) until used for cDNA synthesis.

First Strand cDNA Synthesis of SA-11 dsRNA

Poly A-tailed SA-11 dsRNA (1-2$\mu$g) was lyophilized then strand separated by resuspending in 5 $\mu$l of 90 percent DMSO (Aldrich-Spectrophotometric Grade) and incubating 30 min. at 37° C. Strand separated RNA was added last to complete a 100ul reaction mixture containing: 50mM Tris-HCl (pH 7.5), 10 mM magnesium acetate, 10mM dithiothreitol (DTT), 2 mM dATP, 2 mM dGTP, 2 mM dTTP, 0.2 mM (alpha-[32P])-dCTP (10,000 Ci/mol), 100 $\mu$g/ml oligodT$_{(12-18)}$ (PMB) (used as a primer), 0.6 U/$\mu$l RNasin, and 0.4 U/$\mu$l reverse transcriptase (Life Sciences). The reactants were incubated for 1 hour 37° C, then synthesis was stopped by addition of an equal volume of Tris-buffer saturated phenol. The equeous phase was adjusted to 20 mM EDTA, and 0.2 N NaOH and incubated overnight at 37° C to remove template RNA then neutralized with an equal volume of 0.2 N HCl and applied to a Sephadex G100 column (Pharmacia) to remove salts and unreacted nucleotides. Fractions containing [32P]-labelled SA-11 cDNA were collected and the DNA was concentrated by precipitation with 0.2M NaAc and 2 volumes of ethanol (-20°). Typically, 0.1-0.2 $\mu$g cDNA were synthesized per microgram of template dsRNA. The integrity of SA-11 cDNA was evaluated by electrophoresis on a one percent alkaline agarose gel containing 30 mM NaOH, 2 mM EDTA to remove secondary structures.

Annealing and Second Strand Synthesis of SA-11 CDNA

SA-11 cDNA was resuspended in 20 microliters of 50 mM Tris-HCl (pH 7.5) and 0.1 M NaCl, then heated for 5 min. 65° C followed by 1 hour at 55° C; and slow cooled to promote annealing of complementary cDNA strands. Annealed cDNA was then precipitated by addition of two volumes of ethanol (-20° C).

Annealed cDNA was treated again with reverse transcriptase to render any remaining single-stranded ends double-stranded. 0.1-0.2 μg annealed SA-11 cDNA was added to a reaction mix identical to that used for first strand synthesis without dsRNA. The reaction was carried out for 30 minutes at 42°C then stopped by addition of an equal volume of Tris buffer-saturated phenol. The aqueous phase, after extraction with phenol, was precipitated with 0.2M NaAc and 2 volumes of ethanol (-20°C).

Size Selection of SA-11 Double-Stranded CDNA

$^{32}$P-labelled SA-11 double-stranded cDNA was resolved by electrophoresis on a 1 percent agarose gel containing TBE buffer (89 mM Tris-base, 89 mM boric acid, and 2.5 mM EDTA) using standard methods [Maniatis et al., Molecular Cloning -A Laboratory Manual, Cold Spring Harbor Laboratory, Cold Spring Harbor, New York, (1982). λ DNA digested with endonuclease HindIII and with endonuclease φX DNA digested with endonuclase HaeIII were applied as size markers. The gel was stained with 0.01 percent ethidium bromide (EtBr) to visualize the size markers. $^{32}$P-labelled gene 6 DNA, visualized by autoradiography of the gel on X-ray film (Kodak XAR-5), and migrating in a size range of 1200-1500 base pairs as judged by size markers, was excised from the gel. The gel slice was placed in a dialysis bag containing 0.1X TBE buffer, and the DNA was electroeluted for 4 hours at 100 volts in 0.1X TBE. The eluted DNA was concentrated and purified using an Elutip™-d column (Schleicher and Schuell) following the manufacturers specifications. Purified, size-selected SA-11 cDNA was stored in ethanol (-20°) until further use.

Terminal Transferase Tailing of SA-11 cDNA)

Deoxycytidine nucleotides (dC) were added to the 3' termini of SA-11 double-stranded cDNA (C-tailing) using terminal transferase (PMB). 0.01-0.1μg cDNA was C-tailed according to the manufacturers' recommendations (BRL) in 20 μl reactions using 10 μM $^3$H-dCTP (100,000 Ci/mol). Reactions were allowed to proceed for 3 minutes at 37°C resulting in addition of 10-20 dC residues per DNA 3'-end. Reactions were stopped with an equal volume of Tris buffer-saturated phenol. C-tailed cDNA recovered in the aqueous phase after phenol extraction was precipitated with 0.2 M NaAc and 2 volumes of ethanol (-20° C).

Annealing of C-Tailed SA-11 cDNA with G-Tailed Plasmid DNA and Cloning in E. coli

0.01-0.1 μg C-tailed SA-11 cDNA was suspended in 50 mM Tris (pH 7.5), 0.1M Nacl, and an equal number of picomoles of pUC9, cut with PstI and G-tailed (PMB), was allowed to anneal by heating 5 min at 65° then for 1 hour at 55° before being allowed to slow cool. The annealed cDNA-plasmid was precipitated with 2 volumes of ethanol (-20°).

One hundred μl of E. coli JM83 cells were made competent using CaCl₂ according to standard procedures [Maniatis et al., Molecular Cloning -A Laboratory Manual, Cold Spring Harbor Laboratory, Cold Spring Harbor, New York, (1982)] and were added to lyophilized SA-11 cDNA-pUC9 DNA (0° C). After 1 hour on ice, the cells were heat-shocked for 2 minutes at 42°, incubated in 1 ml of LB (Luria Broth) media for 1 hour at 37° and applied to LB agar plates containing ampicillin (Amp) and the color indicator X-gal. Colonies containing pUC9 metabolize X-gal resulting in a blue phenotype. [Maniatis et al., Molecular Cloning -A Laboratory Manual, Cold Spring Harbor Laboratory, Cold Spring Harbor, New York (1982)]. When the lacZ' sequence of pUC9 is interrupted by the presence of an insert, a white colony results. White colonies were replated and assayed further by colony hybridization.

Example 2

Clones were screened initially by the presence of white colonies on agar plates containing X-gal indicating plasmids which contained SA-11 cDNA sequences. The clones were re-screened by colony hybridization using radiolabeled gene 6 mRNA as a probe. Colonies which hybridized positively to gene 6 mRNA were further characterized by sizing of the insert DNAs and mapping with restriction endonucleases.

Synthesis Of SA-11 mRNA Probe and Purification Of Gene 6 mRNA

$^{32}$P-labelled SA-11 mRNA was synthesized for use as a probe from CsCl-purified SA-11 virus in a 100 $\mu$l reaction containing: 60 mM Tris-HCl (pH8.3), 6 mM Mg(Ac)$_2$, 100 mM NaAc, 2 mM GTP, 0.1mM ATP - (10,000 Ci/mol), 0.1 mM CTP (10,000) Ci/mol), 0.1 mM UTP (10,000 Ci/mol), 1.0 mM S-adenosyl methionine (SAM), 0.6 U/$\mu$l RNasin, and 10 $\mu$g of SA-11 virus. Reactions were carried out for 4 hours at 37° C then stopped by extraction with an equal volume of Tris buffer-saturated phenol. $^{32}$P-labelled mRNA was applied to a Sephedox G100 column to remove unreacted nucleotides. Labelled fractions containing mRNA were pooled and concentrated by precipitation with 0.2M NaAc and 2 volumes of ethanol (-20°C). $^{32}$P-labelled SA-11 mRNA species were resolved by electrophoresis on a 1.5 percent agarose gel containing E buffer - (18 mM Na$_2$HPO$_4$, 2 mM NaH$_2$PO$_4$) and 6 percent formaldehyde as a denaturing agent. Messenger RNA samples were denatured before loading onto the gel by heating for 5 min. at 65° C in E buffer, 6 percent formaldelyde, and 50 percent formamide, then quick cooling on ice. $\lambda$ DNA, digested with HindIII, and $\phi$X DNA, digested with Hae III, were applied to the gel as size markers. The gel was stained with 0.01 percent ethidium bromide (EtBr) to visualize the DNA markers. The mRNA species migrating at a mobility expected for gene 6 mRNA (1350 base pairs) was excised from the gel, the gel slice was inserted into a dialysis bag containing 0.1X TBE buffer, and the mRNA was electroeluted for 4 hours at 100 volts in 0.1X TBE. The eluted mRNA was concentrated and purified using an Elutip (Scheicher and Shuell) following the specifications of the manufacturer. Gene 6 mRNA was stored in ethanol (-20°) until use as a probe for colony hybridization.

Colony Hybridization And Small Scale Plasmid Screening Of SA-11 Gene 6 Cloned DNA

Colonies derived from cloning SA-11 cDNA into E. coli strain JM 83 were screened for the presence of gene 6-specific DNA sequences by colony hybridization using $^{32}$P-labelled SA-11 gene 6 mRNA as a probe. Colony hybridization was carried out by the method of Grunstein and Hogness using standard protocols. Maniatis et al., Molecular Cloning -A Laboratory, Cold Spring Harbor Laboratory, Cold Spring Harbor, New York (1982). Colonies which were identified as hybridizing to gene 6 mRNA by autoradiography of X-ray film (Kodak XAR-5) were amplified by incubation overnight in LB media.

E. coli cells containing gene 6 DNA sequences were pelleted and lysed following the standard procedures of Birnboim et al., [Maniatis et al., Molecular Cloning -A Laboratory Manual, Cold Spring Harbor Laboratory, Cold Spring Harbor, New York, (1982)]. Plasmid DNAs recovered from the cell supernatant were digested with PstI endonuclease to excise insert DNAs. The PstI-cut plasmid DNAs were resolved by electrophoresis on 1 percent agarose gels in TBE buffer along with $\lambda$ DNA cut with HindIII and $\phi$X HaeIII-cut DNA as size markers. A plasmid containing an apparently full-size insert of gene 6 DNA (pAR61) was mapped further with restriction endonucleases. The size of the excised inserts was determined by mobility on agarose gel electrophoresis.

Restriction Endonuclease Mapping Of SA-11 Gene 6 and Cloned DNA

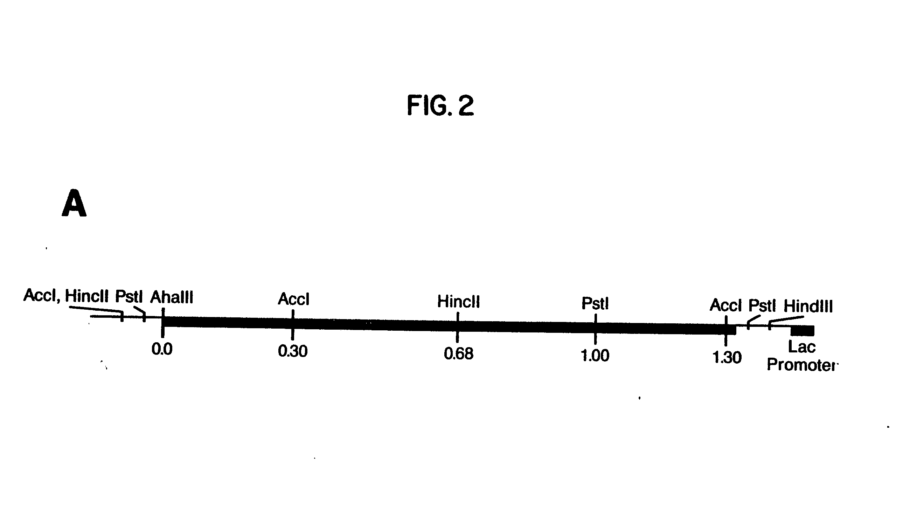

Gene 6 DNA contained in plasmid pAR61 was mapped with restriction endonucleases AhaIII, AccI, HincII, and PstI to better estimate the size of the insert DNA and to compare with published restriction sites for SA-11 gene 6. Estes et al., Nucleic Acids Res. 12, 1875-1887 (1984) and Both et al., J. Virol. 51, 97-101 (1984). The resulting restriction map as illustrated in Fig. 2, confirms the presence of an almost full-size insert of SA-11 cDNA. The orientation of gene 6 DNA in pUC9 (pAR61) as determined by digestion with HincII and AccI, which are contained within the universal cloning site of pUC9 and flank the insert site of gene 6 DNA as well as within the gene 6 sequence. Analysis of the restriction digests by agarose gel electrophoresis showed that gene 6 was inserted in the reverse orientation with respect to the lac promoter.

## Example 3

To confirm that the DNA inserted in pAR61 was SA-11 gene 6, radiolabeled probe was made from gel purified insert DNA and hybridized to a mixture of SA-11 dsRNAs resolved by electrophoresis on an acrylamide gel and blotted onto a GenescreenTM membrane.

### Northern Blot Hybridization Of SA-11 Gene 6 DNA

SA-11 gene 6 cloned DNA was excised from pAR61 with PstI endonuclease then purified by electrophoresis on a 1 percent agarose gel in TBE buffer followed by excision of the gene 6 fragment, electroelution, and recovery by ElutipTM procedures as described above for purification of SA-11 cDNA. Purified gene 6 DNA was $^{32}$P-labelled in a nick translation reaction using ($\alpha$-$^{32}$P)dCTP according to the manufacturers protocol (NEN). Nick-translated gene 6 DNA served as a $^{32}$P-labelled probe for hybridization to gene 6 by a Northern blot technique.

Unlabelled SA-11 dsRNA and $^{32}$P-Cp labelled dsRNA used as a size marker were resolved by electrophoresis on a 5 percent acrylamide gel in TBE buffer. The resolved RNAs were denatured by treatment with 0.1 M NaOH for 15 min. at room temperature followed by washing with sodium phosphate buffer (pH 5.5) using the method of Bellamy et al., J. Virol., 43, 369-378 (1982). The denatured RNAs were then vacuum blotted onto GenescreenTM membrane (NEN) following the procedure of Peferoen, FEBS Letters, 145, 369-372 (1972), using 20Z SSC, 0.025 M Na$_2$HPO$_4$/NaH$_2$PO$_4$ (pH6.5) as the blotting buffer.

Blotted RNAs were hybridized with gene 6 $^{32}$P-labelled nick-translated probe using standard hybridization protocols with 50 percent formamide. Maniatis et al., Molecular cloning -A Laboratory Manual, Cold Spring Harbor Laboratory, Cold Spring Harbor, New York (1982). Hybridization of gene 6 probe to specific SA-11 dsRNA sequences was determined by autoradiography using X-ray film (Kodak XAR-5).

Analysis of the Northern blot showed that the cloned DNA probe hybridized specifically to genome segment 6 confirming that the insert is a cloned DNA copy of gene 6 dsRNA.

## Example 4

The cloned DNA of SA-11 gene 6 was sequenced according to the method of Sanger, using M13 cloning vectors and primers, and according to the method of Maxam and Gilbert as follows.

### Sequencing Of SA-11 Gene 6 Cloned DNA

The termini of SA-11 gene 6 cloned DNA were sequenced by the Maxam and Gilbert chemical method using protocols and reagents supplied by the manufacturer (NEN). The plasmid pAR61 was digested alternately with HindIII or EcoRI in the iniversal cloning site adjacent to the gene 6 insert, then end-labelled with ($\gamma$-$^{32}$P)ATP, degraded chemically with specific reagents, and resolved by electrophoresis on 8 percent or 20 percent acylamide gels using TBE buffer. To extend the terminal sequences, pAR61 was cut with AhaIII or AccI to avoid dG/dC homopolymeric tails and processed in a similar manner.

The internal sequence of gene 6 was determined by Sanger dideoxy sequencing methodology (PMB). Gene 6 was excised with PstI and a 350 base pair (bp) fragment was subcloned into the PstI site of M13 cloning vector mp8 (PMB). A sequence-specific DNA primer (PMB) flanking the PstI site of mp8 was used to initiate synthesis of DNA polymerase reactions containg ($\alpha$-$^{32}$P)-labelled deoxynucleotides (NEN). $^{32}$P-labelled fragments were resolved by acrylamide gel electrophoresis as previously indicated.

The large PstI fragment of gene 6 (1000bp) was sequenced from subclone pAR63, the construction of which is described in Example 5. The large fragment was excised using SmaI and PstI restriction enzymes and subcloned into the SmaI, Pst I sites of mp8. mp8 was then recut with PstI and a series of deletions were constructed using the exonuclease activity of T4 DNA polymerase using the Cyclone System™ of International Biotechnologies Incorporated (IBI). mp8 subclones containing the gene 6 DNA deletions were sequenced by standard Sanger procedures (PMB).

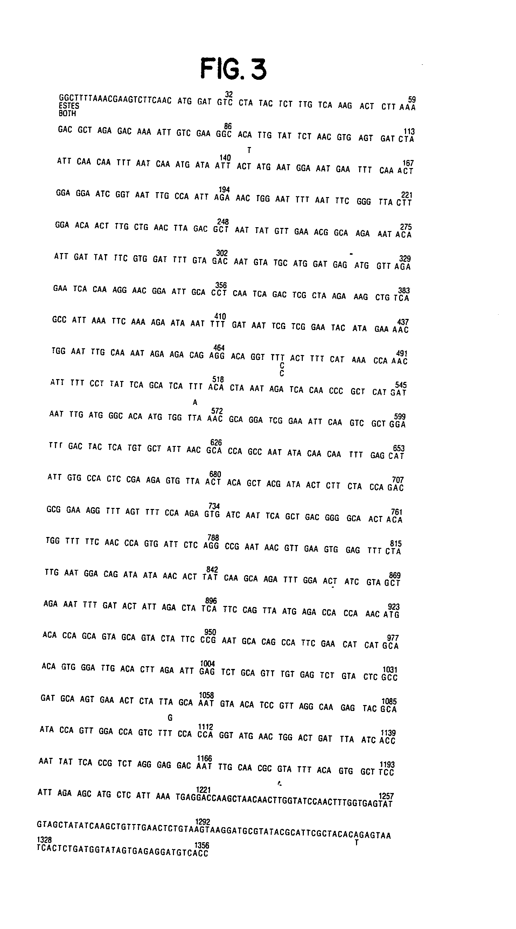

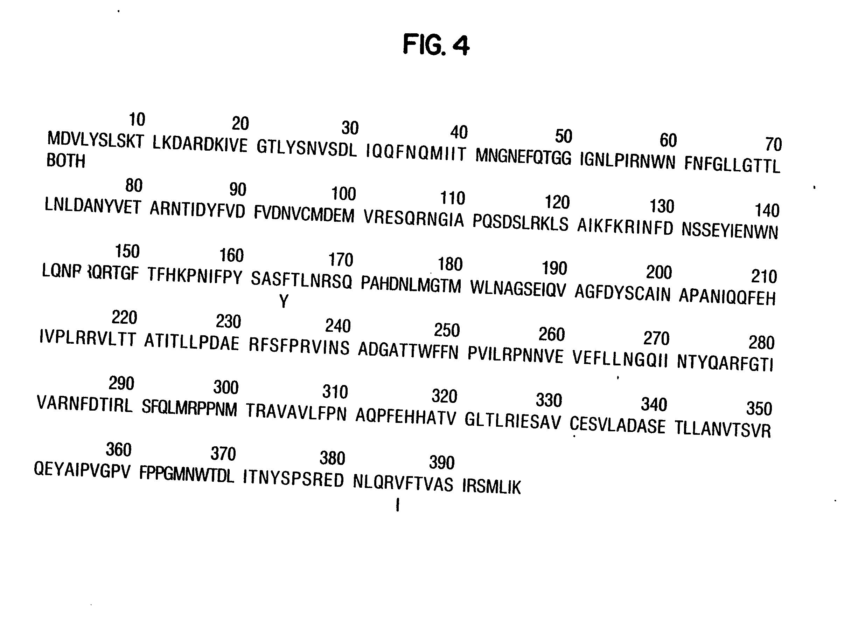

Analysis of the DNA sequence of the SA-11 cloned DNA, as provided in Figure 3, showed that the entire gene 6 sequence was present except for the final 32 base pairs of the 3'-nontranslated region. Comparison of the sequence of Figure 3 to other published sequences for SA-11 gene 6 [Estes et al., Nucleic Acids Res ., 12, 1875-1887 (1984); and Both et al., J . Virol. 51, 97-101 (1984)] showed only two base pair differences when compared to the sequence of Estes, et al. and five differences when compared to Both, et al. As shown in Fig. 4, the inferred amino acid sequence is indentical to the sequence of Estes, et al. and two amino acid differences with Both, et al., both differences being highly conserved.

## Example 5

### Subcloning Of SA-11 Gene 6 DNA For Expression In E. coli

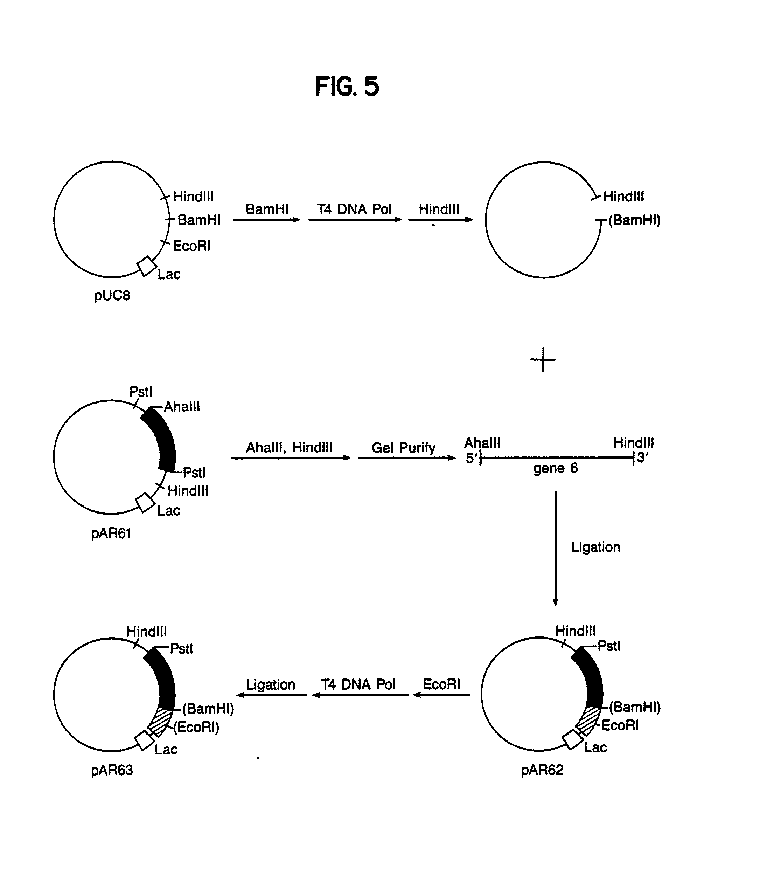

As illustrated in Figure 5, gene 6 DNA was excised from pAR61 using restriction nucleases AhaIII and HindIII which allowed for removal of 5'-terminal homopolymeric dG tails flanking the coding region of the gene. The gene 6 insert DNA was purified by gel electrophoresis, electroelution, and Elutip™ methodologies described above. The insert DNA was joined to pUC8 using DNA ligase according to the suppliers specifications (BRL). The plasmid pUC8 had been digested with Bam HI then made blunt ended using DNA polymerase Klenow fragment (BRL specifications) followed by digestion with EcoRI prior to ligation. The ligated DNA was cloned into E. coli strain JM 105 using a standard CaCl$_2$ transformation procedure. Maniatis et al., Molecular Cloning -A Laboratory Manual, Cold Spring Harbor Laboratory, Cold Spring Harbor, New York, (1982). The resulting construction, designated, pAR62 placed the gene 6 DNA in the forward orientation with respect to the lac promoter in pUC8 and in frame with the N-terminal 6 amino acids of lacZ', allowing for expression as a fusion protein.

The resulting fusion protein, the amino acid sequence of which is given in Table 1, contains 15 additional amino acids preceeding the initiator methionine of native VP6. These are derived from the N-terminal amino acids of lacZ', and the universal cloning site of pUC8 and the 5'-nontranslated region of gene 6 (MTMITNSRETKSSTW).

## TABLE 1

```
        -10       +1        10        20        30        40
      MTMIT NSRETKSSTW MDVLYSLSKT LKDARDKIVE GTLYSNVSDL IQQFNQMIIT

        50        60        70        80        90
      MNGNEFQTGG IGNLPIRNWN FNFGLLGTTL LNLDANYVET ARNTIDYFVD

       100       110       120       130       140
      FVDNVCMDEM VRESQRNGIA PQSDSLRKLS AIKFKRINFD NSSEYIENWN

       150       160       170       180       190
      LQNRRQRTGF TFHKPNIFPY SASFTLNRSQ PAHDNLMGTM WLNAGSEIQV

       200       210       220       230       240
      AGFDYSCAIN APANIQQFEH IVPLRRVLTT ATITLLPDAE RFSFPRVINS

       250       260       270       280       290
      ADGATTWFFN PVILRPNNVE VEFLLNGQII NTYQARFGTI VARNFDTIRL

       300   .   310       320       330       340
      SFQLMRPPNM TRAVAVLFPN AQPFEHHATV GLTLRIESAV CESVLADASE

       350       360       370       380       390
      TLLANVTSVR QEYAIPVGPV FPPGMNWTDL ITNYSPSRED NLQRVFTVAS
```

IRSMLIK

An out-of-frame mutation of pAR62, designated pAR63, was created by digestion of pAR62 with Eco RI, blunt-ending with DNA polymerase Klenow fragment, ligation with DNA ligase to form the plasmid pAR63, and cloning in JM 105 as indicated above. The reverse orientation of pAR62 was generated by excising gene 6 from pAR62 with EcoRI and HindIII nucleases and inserting into the EcoRI, HindIII sites of pUC9 by procedures described above to form the plasmid pAR62r. Restriction maps of pUC8 and pUC9, used for mapping and orientation of cloned SA-11 gene 6 DNA, were as provided by Pharmacia Molecular Biologicals.

Example 6

In Vitro Expression Of SA-11 Gene 6 Cloned DNA

Expression of gene 6 cloned DNA in an E coli cell-free transcription/translation system (Amersham) was evaluated using plasmids pAR62, pAR63, pAR62r, and gel purified gene 6 DNA. λ DNA and pUC9 DNA were also included as controls. Manufacturers instructions (Amersham) were followed except that only half of the recommended reaction volumes were used.

35S-L-methionine containing proteins were resolved by electrophoresis on a 10 percent SDS-acrylamide gel with a 4 percent stacking gel according to methods described by Laemmli, Nature, 227, 680-685 (1970). Exposure of labelled proteins was enhanced by treating the gel with ENhance (NEN) and drying prior to placing under X-ray film (Kodak XAR-5) with a Quanta IIITM intensifying screen at -70° C.

As revealed by SDS-acrylamide gel electrophoresis of the recombinant protein products, two proteins of approximately 38 kilodaltons (38K) and a protein of 27K were synthesized using plasmids pAR63, pAR62r, and purified gene 6 DNA. These proteins are smaller than native VP6 (41K) and are probably coded for by mRNAs initiating at transcription start sites within the gene 6 sequence which are recognized by E. coli RNA polymerase. In addition to the 38K and 27K proteins, pAR62 expressed a 44K protein, indicating the successful fusion of VP6 to the N-terminus of lacZ'.

## Example 7

The products of SA-11 gene 6 derived from plasmid pAR62 by in vitro expression in E. coli cell-free system were assayed for immune reactivity with SA-11 antibodies by immune precipitation. Immune precipitation of SA-11 virus and pAR62 gene 6 protein products were compared respectively using no antibody, normal serum, antiserum to SA-11 virus, and mono-specific antiserum to purified VP6.

## Immune-Precipitation Of SA-11 Gene 6 Proteins Expressed In Vitro

35S-labelled proteins expressed in vitro in an E. coli cell-free system using plasmid pAR62 and 35S-labelled SA-11 virus were assayed for their ability to precipitate with SA-11 specific antiserum and formalin-inactivated Staph A (Miles). Polyclonal antiserum to SA-11 inner capsid particles derived from guinea pigs - (Abbott Laboratories) and antiserum derived from guinea pigs to VP6 protein purified by SDS-acrylamide gel electropharesis (VP6 monospecific serum obtained from M.K. Estes) were reacted with 35S-labelled SA-11 virus and 35S-labelled in vitro protein products of pAR62 according to procedures indicated by Mason, et al., Virol. 33, 1111-1121 (1980). Immune-complexed proteins were bound to Staph A and unbound proteins were removed as shown by Mason, supra. Staph A-precipitated proteins were resolved by electrophoresis on a 10 percent SDS-acrylamide gel with a 4%-stacking gel in gel buffer [Laemmli Nature, 227, 680-685 (1970).]

The results demonstrate that viral VP6 protein (41K) and all the gene 6 products expressed in E. coli extracts (44K, 38K, 27K) were immune-precipitated with both antiserum to SA-11 virus and with mono-specific antiserum to purified VP6. By contrast, viral protein and E. coli-expressed products were not precipitated in the absence of serum and only weakly by normal serum. The smaller protein species (27K) as well as a minor protein of 24K have the greatest immune-reactivity, suggesting that truncated protein products of gene 6 DNA expressed in E. coli would be potentially useful as antigenic reagents for diagnostic assays such as Rotazyme ® .

## Example 8

Plasmid vectors containing SA-11 gene 6 DNA assayed for expression in vitro (pAR62, pAR63) were transformed into E. coli strain JM105 (Pharmacia Molecular Biologicals) and assayed for expression in vivo using the Rotazyme ® assay. (available from Abbott Laboratories Diagnostics Division, Abbott Park, North Chicago, Illinois).

## Expression Of SA-11 Gene 6 Cloned DNA In Vivo In E. coli

Plasmids pUC8, pAR62, and pAR63 were transformed into E. coli strains JM 105, JM 103, and WM 6 using standard $CaCl_2$ transformation methodologies, Laemmli, Nature, 227, 680-685 (1970). E. coli-containing plasmids were grown of in 25 ml cultures of LB at 37° C to an optical density ($OD_{600}$) of 1.5. Cells were harvested by centrifugation for 10 min., at 3000g (4° C) and lysed according to the following procedure. Pelleted cells were resuspended in 0.75 ml of Buffer A [25% sucrose, 50 mM Tris-HCl (pH 8.0)]. The cells were transferred to a 5 ml polystyrene tube and 0.15 ml of Buffer B [50 mM Tris-HCl (pH 8.0), 10 mM EDTA] containing 5 mg/ml lysozyme (Sigma) was added. This mixture was incubated for 10 minutes on ice.

Three tenths of a milliliter of buffer C [0.25 M Tris-HCl (pH 8.0), 0.25 M EDTA] was added to the cells and incubated as before. Following the second incubation, 1.2 ml of Buffer B containing protease inhibitors (4 μM PMSF, 1.2% Aprotinin as available from Sigma) were added. The cells were sonicated on ice for 15-30 seconds or until lysed and stored at -20° C.

## Immune-Reactivity Of SA-11 Gene 6 Proteins Expressed in E. coli

The antigenicity of gene 6 protein products expressed in E. coli strain JM 105 was evaluated for plasmids pUC8, pAR62, and pAR63 using the Rotazyme$^©$ assay (Abbott Laboratories). Two hundred microliters of sonicated lysate for each sample, prepared as indicated above, was incubated with a polystyrene bead coated with guinea pig anti-rotavirus antibody and developed according to the manufacturers' conditions for the Rotazyme$^©$ assays.

Quantitative results of the assay as presented in Table 2, show that both plasmids are expressed in E. coli relative to a pUC8 control, but that pAR62 yields a higher antigenic response, presumably due to expression of VP6 as a fusion protein.

TABLE 2

| Sample | $OD_{492}$ |
|---|---|
| Buffer | 0.08 |
| 10 ng SA-11 | 1.61 |
| 100 ng SA-11 | 2.76 |
| pUC8 | 0.12 |
| pAR62 | 2.76 |
| pAR63 | 1.00 |

## Example 9

## Expression of SA-11 gene 6 cloned DNA in an E. coli protease-deficient strain (WM 6).

The antigenicity of SA-11 gene 6 protein products expressed in E. coli strains JM103 and WM 6 was also evaluated for plasmids pUC8, pAR62, and pAR63 using the Immun-Blot™ procedure (Bio Rad). 100 μl sonicated lysates were applied to a nitrocellulose filter pre-wet with TBS (20 mM Tris-HCl (pH7.5); 0.5 M NaCl) and mounted in a Minifold™ filtration manifold (Schleicher and Schuell). SA-11 virus in the amounts of 20 ng, 50 ng, 100 ng, 200 ng, 300 ng, and 400 ng SA-11 virus was also applied as a control. The dried membrane was then immersed sequentially in blocking buffer, first antibody buffer containing a 1/2000 dilution of guinea pig anti-rotavirus polyclonal antiserum (Abbott Laboratories), second antibody, and color development reagent following the manufacturers protocol (Bio Rad). To prevent fading of color, nitrocellulose sheets were mounted betwen glass plates and stored in the dark.

Levels of expression of pAR63 products were seen to be similar in JM 103 and WM 6 comparing intensities of color reactions of spotted antigenic protein products. By contrast, the expression of pAR62 products was enhanced in WM 6 but diminished in JM 103 relative to pAR63. This result suggests that the lacZ'-VP6 fusion protein product is protease sensitive. In addition, gene 6 protein products of pAR62 were observed to be much more antigenic than those of pAR63 in WM 6. This difference may again be attributed to the unique structure of the lacZ'-VP6 fusion protein.

Protein products from gene 6 DNA expression in JM 103 and WM 6 were resolved by electrophoresis on an SDS-acrylamide gel and probed with mono-specific antisera to purified VP6 using a Western blotting procedure.

Western Blot Assay Of SA-11 Gene 6 Proteins Expressed In E. coli

Thirty microliters of sonicated lysates from JM 103 and WM 6 containing plasmids pUC8, pAR62, and pAR63 were applied to a 10 percent SDS-acrylamide gel with a 4 percent stacking gel, Laemmli, Nature, 227, 680-685 (1970). SA-11 virus in the amounts of 100 ng and 200 ng was also applied as a standard. The resolved proteins were electroblotted from the gel onto a nitrocellulose sheet using a Trans-Blot™ Cell (Bio Rad) and 20 mM Tris base, 150 mM glycine, 20 percent methanol buffer overnight at 150 milliamps (mA). The dried nitrocellulose sheet containing transferred proteins was assayed for the presence of VP6-specific antigens using the same reagents and protocols as described in Example 9 for the Immun-Blot™ procedure except that a 1/200 dilution of guinea pig anti-VP6 monospecific antiserum (obtained from M.K. Estes) was used as the first antibody. To prevent fading of color, nitrocellulose sheets were mounted between glass plates and stored in the dark.

The 38K and 27K proteins expressed in vitro using pAR63 are also seen in vivo for both JM 103 and WM 6 strains, although both proteins appear to be made in greater amount in WM6, as revealed by Western blot assay. A 12K protein was expressed from pAR62 in JM 103. By contrast, a 44K protein product was expressed in WM 6 similar to that seen in vitro as well as a number of smaller species. These data confirm the protease-sensitive structure of the lacZ'-VP6 fusion protein.

It is interesting that the relative intensity of immune reactions is less for the products of pAR63 compared to pAR62 in WM 6 by immune-spot assay, but the opposite was observed by Western blotting assays. Western blotting assays are particularly sensitive in the detection of antigenicity arising from the primary structure of antigens, i.e. the amino acid sequence, while immune-spot assays are useful for detection of conformation. This result implies that most of the enhancement in immunoreactivity of pAR62 products relative to pAR63 products is due to unique conformational features of the lacZ'-VP6 fusion protein.

Example 10

The fusion protein and fragments according to the present invention may be employed in a solid phase assay for the presence of antibodies, such as the Rotazyme ® assay described in Example 8. In such an assay, particles are coated, with antibodies e.g. guinea pig, specific for major capsid protein and then with fusion protein or fragments thereof before being trapped in a glass filter matrix. A stool sample diluted with buffer or a serum sample is passed through the matrix. If antibodies against the major capsid are present in the sample, they are detected by exposure to an alkaline-phosphatase-conjugated second antibody specific for major capsid protein. By passing a solution of the second antibody through the matrix, the second antibody binds to any major capsid protein therein.

After washing an incubation of the matrix with the substrate formulation according to the present invention, any reaction is stopped by washing excess substrate from the matrix.

In the presence of major capsid protein, alkaline phosphatase-conjugated antibody bound to the major capsid protein reacts with the substrate formulation to produce a dark, blue-black spot. In the absence of major capsid protein, the conjugated antibody is washed away and no spot is formed.

In a solid phase assay, a plurality of substantially spherical solid particles, having an average diameter of from about 0.1 to about 5 microns, are immobilized on a porous matrix of fibrous material. The fibrous material may be formed from glass, polystyrene-coated glass, cellulose, nylon or other fibrous materials known to interact with particles and to immobilize them. The particles may be composed of polystyrene, polymethacrylate, polypropylene, latex, polyacrylonitrile, polycarbonate or similar materials which have a surface capable of holding a substance to be analyzed

For a rotavirus assay, microparticles may be prepared by adding carboxylate-modified polystyrene microparticles (commercially available from Seragen, Indianapolis, Indiana) a buffer at pH 4.75 and anti-major capsid protein antibody solution. The solution is stirred before adding ethyl 3(3-dimethyl)aminopropyl carbodiimide HCl (EDAC) at a conclusion of 0.5 mg/ml H₂O. The solution is stirred overnight at 2-8° C, after which the microparticles are isolated by centrifugation, washed twice with Tween-20 solution, and resuspended in phosphate buffered saline 0.1M KH₂PO₄; and 0.15 M NaCl at pH 7.2). After resuspension in phosphate buffered saline (PBS), the particles are stored at 2-8° C for subsequent use.

Antibody-enzyme conjugates may be prepared according to the procedure of Kearney et al., Immunology, 123, 1548 (1979) from anti-major capsid protein monoclonal antibodies. Alkaline phosphatease may be obtained from Boehringer Mannheim GmbH, Indianapolis, Indiana.

Example11

The substrate formulation according to the present invention may be employed in an enzyme-linked immunosorbent assay (ELISA) for the purpose of determining the presence of an antibody against a rotavirus in a blood sample. In such an assay, an antigen according to the present invention is spotted on nitrocellulose. The nitrocellulose is incubated with a patient sample followed by incubation with an alkaline phosphatase-conjugated antibody against human antibody. Subsequent incubation of the nitrocellulose with the substrate formulation according to the present invention results in the formation of a blue-black colored spot if an antibody specific for the spotted antigen is present in the sample. If no such antibody is present, no spot forms.

Example 12

Isolates from transformed E. coli cells according to the present invention may be column purified and employed to produce antisera or as a vaccine.

Antisera may be specifically produced by immunizing rabbits with injections of purified fusion protein according to the present invention as follows. The first inoculation contains the antigen with Freund's complete adjuvant. Succeeding inoculations contained the antigens and 0.25 ml of Freund's incomplete adjuvant. The animals are bled to obtain sera. Polyclonal antibodies may be isolated from the sera by affinity chromatography.

A vaccine solution according to the present invention may be prepared by suspending a fusion protein according to the present invention in an immunologically acceptable diluent adjuvant and carrier. Initial and booster injections of vaccine solution may be used to confer immunity.

Example 13

Monoclonal antibodies according to the present invention may be produced according to the procedure of Greenberg et al., Infect. Immun., 39, 91-99 (1983) with the substitution of a solution of fusion protein according to the present invention for the rotavirus concentrate employed therein. Greenberg et al., is incorporated by reference herein.

Basically, monoclonal antibodies are produced by injecting mice with immunizing doses of fusion protein, as described in Example 12. Spleens are removed from the immunized animals, and spleen cells are fused to myeloma cells (e.g. NS-1 cells) using a fusogen, such as polyethylene glycol. Hybridoma cells producing monoclonals are selected for in HAT medium. Monoclonal antibodies specific for lacZ' VP6 fusion protein may be isolated by affinity chromatography from media in which such hybridomas have been cultured.

Although the present invention is described in terms of a preferred embodiment, it is understood that modifications and improvements will occur to those skilled in the art. For example, a lac Z'-VP6 fusion protein expressed in E. coli could be synthesized in large quantities at a low cost relative to production of SA-11 rotavirus in mammalian tissue culture. Such a recombinant antigen could may also be modified by site-specific mutagenisis providing a reagent with superior antigenic properties such as greater sensitivity or a broader spectrum of detection.

Accordingly, it is intended that the appended claims include all such equivalent variations which come within the scope of the invention as claimed.

**Claims**

1. A fusion protein more active than the native major inner capsid protein in a test of antigenic reactivity and selected from the group consisting of:
    (a) a lacZ gene product-rotavirus major inner capsid fusion protein;

(b) a lacZ gene product-rotavirus major inner capsid fusion protein further comprising a 5'-noncoding region of the lac Z gene;

(c) a fusion protein having an amino acid sequence as set forth in Table 1; and

(d) a fragment of (a), (b) or (c).

2. A nucleic acid encoding a fusion protein as recited in Claim 1 wherein said nucleic acid is expressed in a host cell comprising a protease deficient E. coli strain.

3. The host cell as recited in Claim 2 wherein the host cell is E. coli strain WM 6.

4. An assay for detecting the presence of human antibodies against rotavirus in a sample comprising the steps of:

exposing a test solution of a sample of antibody-containing material in a test solution to a fusion protein as recited in Claim 1, the fusion protein being bound to a support;

introducing anti-human antibody associated with a reporter group into the test solution; and

detecting the presence of the reporter group bound to the support.

5. A vaccine comprising an immunologically acceptable diluent, adjuvant or carrier and a fusion protein as recited in Claim 1.

6. A method for raising a polyclonal antibody against a fusion protein as recited in Claim 1 comprising the steps of:

immunizing an animal with an effective amount of fusion protein as recited in Claim 1; and

isolating anti-VP6 antibodies from serum of the animal.

7. A polyclonal antibody produced according to the method as recited in Claim 6.

8. A method of producing a monoclonal antibody against a fusion protein as recited in Claim 1 comprising the steps of:

immunizing an animal with an effective amount of fusion protein as recited in Claim 1;

fusing antibody-producing cells from the animal with myeloma cells;

isolating a hybridoma in a culture medium producing a monoclonal antibody against VP6; and

purifying the monoclonal antibody from the culture medium.

9. A monoclonal antibody produced according to the method as recited in Claim 8.

FIG. 1

## FIG. 2

**A**

AccI, HincII PstI AhaIII     AccI          HincII          PstI          AccI PstI HindIII

0.0          0.30          0.68          1.00          1.30     Lac Promoter

0 235 754

# FIG. 3

```
GGCTTTTAAACGAAGTCTTCAAC ATG GAT GTC CTA TAC TCT TTG TCA AAG ACT CTT AAA
ESTES
BOTH
                                         T
GAC GCT AGA GAC AAA ATT GTC GAA GGC ACA TTG TAT TCT AAC GTG AGT GAT CTA

ATT CAA CAA TTT AAT CAA ATG ATA ATT ACT ATG AAT GGA AAT GAA TTT CAA ACT

GGA GGA ATC GGT AAT TTG CCA ATT AGA AAC TGG AAT TTT AAT TTC GGG TTA CTT

GGA ACA ACT TTG CTG AAC TTA GAC GCT AAT TAT GTT GAA ACG GCA AGA AAT ACA

ATT GAT TAT TTC GTG GAT TTT GTA GAC AAT GTA TGC ATG GAT GAG ATG GTT AGA

GAA TCA CAA AGG AAC GGA ATT GCA CCT CAA TCA GAC TCG CTA AGA AAG CTG TCA

GCC ATT AAA TTC AAA AGA ATA AAT TTT GAT AAT TCG TCG GAA TAC ATA GAA AAC

TGG AAT TTG CAA AAT AGA AGA CAG AGG ACA GGT TTT ACT TTT CAT AAA CCA AAC
                                             C
                                             C
ATT TTT CCT TAT TCA GCA TCA TTT ACA CTA AAT AGA TCA CAA CCC GCT CAT GAT
                              A
AAT TTG ATG GGC ACA ATG TGG TTA AAC GCA GGA TCG GAA ATT CAA GTC GCT GGA

TTT GAC TAC TCA TGT GCT ATT AAC GCA CCA GCC AAT ATA CAA CAA TTT GAG CAT

ATT GTG CCA CTC CGA AGA GTG TTA ACT ACA GCT ACG ATA ACT CTT CTA CCA GAC

GCG GAA AGG TTT AGT TTT CCA AGA GTG ATC AAT TCA GCT GAC GGG GCA ACT ACA

TGG TTT TTC AAC CCA GTG ATT CTC AGG CCG AAT AAC GTT GAA GTG GAG TTT CTA

TTG AAT GGA CAG ATA ATA AAC ACT TAT CAA GCA AGA TTT GGA ACT ATC GTA GCT

AGA AAT TTT GAT ACT ATT AGA CTA TCA TTC CAG TTA ATG AGA CCA CCA AAC ATG

ACA CCA GCA GTA GCA GTA CTA TTC CCG AAT GCA CAG CCA TTC GAA CAT CAT GCA

ACA GTG GGA TTG ACA CTT AGA ATT GAG TCT GCA GTT TGT GAG TCT GTA CTC GCC

GAT GCA AGT GAA ACT CTA TTA GCA AAT GTA ACA TCC GTT AGG CAA GAG TAC GCA
                              G
ATA CCA GTT GGA CCA GTC TTT CCA CCA GGT ATG AAC TGG ACT GAT TTA ATC ACC

AAT TAT TCA CCG TCT AGG GAG GAC AAT TTG CAA CGC GTA TTT ACA GTG GCT TCC
                                                     ι
ATT AGA AGC ATG CTC ATT AAA TGAGGACCAAGCTAACAACTTGGTATCCAACTTTGGTGAGTAT

GTAGCTATATCAAGCTGTTTGAACTCTGTAAGTAAGGATGCGTATACGCATTCGCTACACAGAGTAA
                                                                  T
TCACTCTGATGGTATAGTGAGAGGATGTCACC
```

## FIG. 4

MDVLYSLSKT LKDARDKIVE GTLYSNVSDL IQQFNQMIIT MNGNEFQTGG IGNLPIRNWN FNFGLLGTTL
BOTH

LNLDANYVET ARNTIDYFVD FVDNVCMDEM VRESQRNGIA PQSDSLRKLS AIKFKRINFD NSSEYIENWN

LQNP‍RQRTGF TFHKPNIFPY SASFTLNRSQ PAHDNLMGTM WLNAGSEIQV AGFDYSCAIN APANIQQFEH
Y

IVPLRRVLTT ATITLLPDAE RFSFPRVINS ADGATTWFFN PVILRPNNVE VEFLLNGQII NTYQARFGTI

VARNFDTIRL SFQLMRPPNM TRAVAVLFPN AQPFEHHATV GLTLRIESAV CESVLADASE TLLANVTSVR

QEYAIPVGPV FPPGMNWTDL ITNYSPSRED NLQRVFTVAS IRSMLIK
I

0 235 754

# FIG. 5